# EUROPEAN PATENT APPLICATION

(11) **EP 0 611 552 A1**
(43) Date of publication of application: **24.08.1994**
(21) Application number: 94250020.8
(22) Date of filing: 04.02.1994
(51) Int. Cl.: A61B 17/06

(54) **Surgical needle**

(30) Priority: 13.02.1993 DE 4304740
(71) Applicant: ETHICON INC., Somerville, New Jersey 08876-0151 (US)
(72) Inventor: Brunken, Dieter, D-24641 Hüttblek (DE)
(74) Representative: UEXKÜLL & STOLBERG

(57) **Abstract**

The invention relates to a surgical needle (2) with a suture material or thread (4) secured to the end opposite the puncture tip (6), which is characterised in that the surfaces of the puncture tip (6) and of the zone (8) of the needle (2) adjoining it are matt-finished black or coloured black, continuously or with small breaks, by chemical or electrolytic means or by a covering, while the surface of the remaining section (10) of the needle (2), which can constitute up to ca. 50% of its length, consists of bare or untreated metal, up to the thread attachment (12).

## Description

This patent application is a patent of addition to the principal patent DE 42 08 242.0.

The invention relates to a surgical needle with a suture material or thread secured to the end opposite the puncture tip.

Such surgical needles or sewing needles are generally known and in most cases consist of a corrosion-resistant metal, preferably of chrome-nickel steel. For small surgical operations bare needles or those which have not been surface-treated are used. For larger surgical operations, however, coloured or matt-finished needles have recently been used, as described, for example, in U.S. Patent No. 4,959,068. This colouring or matt-finishing is achieved either by chemical means through pickling or etching, or electrolytically using suitable anodic or cathodic treatment, optionally with reversion of polarity or using alternating current. A special form of pickling is also the so-called dipping or matt-dipping. In some cases stoving lacquers are also used for colouring or matt-finishing. As a result of the so-called colouring or matt-finishing of the surgical needle, the needle does not reflect under strong OP light and irritate the operator, an advantage particularly with larger operations.

Despite the popularity of these coloured or matt-finished needles, it has been proved a disadvantage that, particularly with needles which are coloured or matt-finished with shades of yellow to dark red and brown the operator can no longer precisely recognize the puncture area of the needle, i.e. the needle tip and the area adjacent to it, in the similarly coloured tissue or operative field, which makes the precise positioning of the suture difficult, not only in microsurgery but also in surgical operations generally.

Known from U.S. Patent No. 3,840,015 is a surgical needle whose tip is provided with a photoluminescent covering which, when irradiated by a suitable source, is excited to light up. Otherwise the needle is bare or untreated at the surface. The photoluminescent covering does in fact improve the visibility of the needle tip, but does, however, require the use of an additional radiation source. Furthermore, such a covering is not unproblematical with regard to its toxicity. The untreated part of the needle, which, in the embodiment described, constitutes the largest part of its surface, can lead to undesirable and irritating reflexes.

The object of the invention is to overcome the disadvantages mentioned and to provide a surgical needle which demonstrates the advantage of the coloured or matt-finished needle, i.e. absence of dazzle, and at the same time permits a precise insertion of the needle into the corresponding tissue.

To achieve this object, a surgical needle of the design mentioned at the beginning is suggested, which is developed so that the surface of the puncture tip and of the zone of the needle adjoining the latter is matt-finished black or coloured black, continuously or with small breaks, by chemical or electrolytic means or by a covering, while the surface of the remaining section of the needle, which can constitute up to ca. 50% of its length, consists of bare or untreated metal up to the thread attachment.

The surgical needle according to the invention differs from the subject of the principal patent DE 42 08 242.0 in that the puncture tip and the zone of the needle adjoining it do not consist of metal which is bare or has not been surface-treated, but that this does apply here to the remaining part of the needle up to the thread attachment, whilst the tip zone is matt-finished black or coloured black. It has been shown in both cases that it is still possible to work dazzle-free with such a needle, which is a departure from the previous view that the entire needle has to be matt-finished or coloured. With the inventive surgical needle the tip zone clearly stands out from the tissue in the operative field, although it does not reflect any light, as its black colour stands in marked contrast to the generally red background. The operator can, in particular, recognise the needle readily when it emerges from the tissue. An additional advantage is that the rear section of the needle up to the thread attachment is particularly striking due to its bare or metallic surface. The transitional zone between the needle and the thread, the thread being in most cases darkly coloured (green, blue etc.), is thus better recognisable. This ensures that the operator can grip the needle surely with forceps or a gripper, without running the danger of inadvertently gripping the thread and thus damaging it.

The section which is bare or has not been surface-treated preferably accounts for less than ca. 25% of the needle's length, which value also depends on the size and the length of the needle and, seen in absolute terms, can be 4 to 10 mm in particular.

Instead of a continuous black matt-finishing or black colouring of the tip zone of the needle, the latter can also be partially black matt-finished or black coloured, having small breaks, whereby this coloured or matt-finished zone in particular can be matt-finished or coloured in annular or speckled form; the intermediate spaces left blank in the zone which is only partially matt-finished or coloured are preferably to be no larger than 1 to 2 mm.

The invention will be explained in more detail below with the help of drawings; these show:

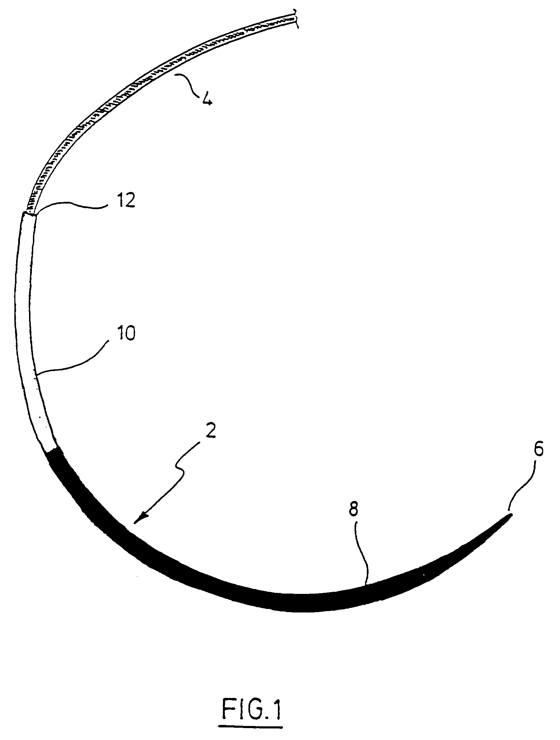

- Figure 1: a magnified representation of the surgical needle according to the invention with a continuously matt-finished or coloured zone;
- Figure 2: a representation analogous to Figure 1 with a zone matt-finished or coloured only bandwise in annular form;
- Figure 3: a representation analogous to Figure 2 with a zone matt-finished or coloured in speckled form.

The needle numbered 2 is a normal semi-circular round-bodied needle; the needle can, however, have any desired form and be, for example, a blunt round-bodied needle, a cutting needle or a spatula needle. Located at one end of the needle is the puncture tip 6, which can also be designed as a microtip, while a thread attachment 12 with the thread 4 is provided at the opposite end of the needle. The tip 6 and the zone 8 adjacent to it, which constitutes ca. more than 50% of the needle length, is continuously matt-finished black or coloured black, chemically or electrolytically or by a covering, whilst the remaining part 10 is bare or has not been surface-treated.

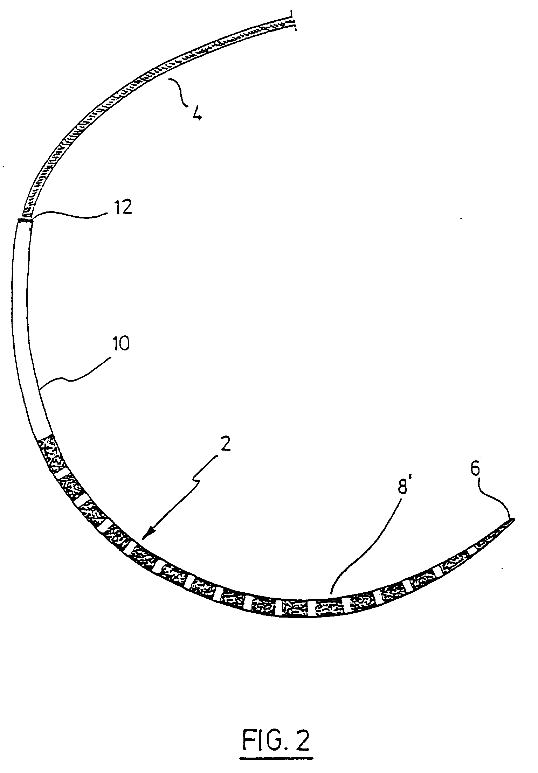

In the representation shown in Figure 2, the matt-finished black or coloured black area 8' is matt-finished or coloured in annular form, whereby the matt-finished or coloured rings, which can be of any width, are ca. 1 to 2 mm apart. This distance is sufficient to avoid a dazzle effect but, on the other hand, also means that the further contour of the needle can be more easily recognized in the operative field than with a continuous black matt-finishing or coloration.

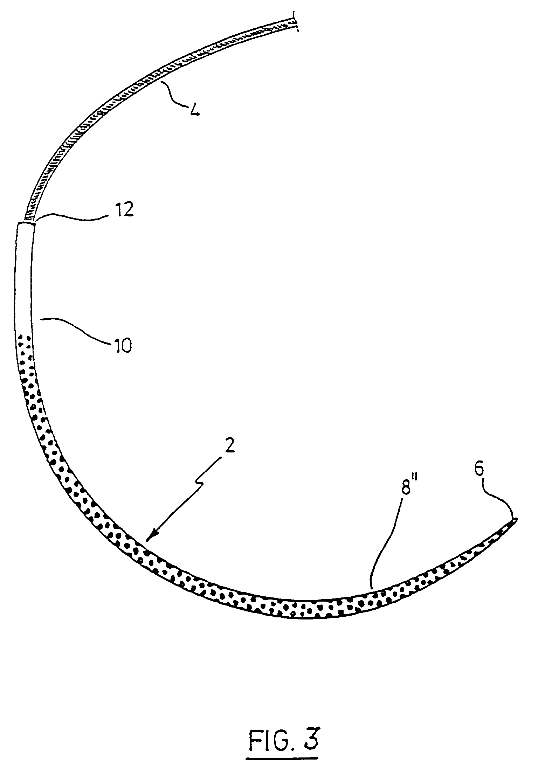

In the case of the needle shown in Figure 3, the matt-finished black or coloured black zone 8'' is speckled, whereby the distances between the coloured or matt-finished speckles are likewise not to exceed 1 to 2 mm.

With all the needles shown, the colour of the matt-finished or coloured zone 8 is black, which is also to be understood to mean another very dark colour that has the same effect as black, namely to largely avoid any reflection of light.

## Claims

1. Surgical needle with a suture material or thread attached to the end opposite the puncture tip, **characterised in that** the surface of the puncture tip (6) and of the zone (8) of the needle (2) adjoining it is matt-finished black or coloured black, continuously or with small breaks, by chemical or electrolytic means or by a covering, while the surface of the remaining section (10) of the needle (2), which can constitute up to ca. 50% of its length, consists of bare or untreated metal up to the thread attachment (12).

2. Needle according to claim 1, **characterised in that** the section (10) of the needle (2) which is bare or has not been surface-treated accounts for less than 25% of the needle length.

3. Needle according to claim 1, **characterised in that** the section (10) which is bare or has not been surface-treated amounts to 4 to 10 mm, measured from the thread attachment (12).

4. Needle according to claim 1, **characterised in that** the zone (6, 8) of the needle (2) which is matt-finished black or coloured black with small breaks is matt-finished or coloured in annular form (8') or in speckled form (8''), and is bare or has not been surface-treated between the matt-finished or coloured areas.
